# EUROPEAN PATENT SPECIFICATION

(11) **EP 3 555 031 B1**
(45) Date of publication and mention of the grant of the patent: **22.04.2026**
(21) Application number: 17835829.7
(22) Date of filing: 18.12.2017
(51) Int. Cl.: C07C 29/132, C07C 31/20

(54) **METHOD FOR STABILIZATION OF GLUCOSE FEED IN THE PRODUCTION OF GLYCOLS**
VERFAHREN ZUR STABILISIERUNG DER GLUCOSEZUFUHR BEI DER HERSTELLUNG VON GLYKOLEN
PROCÉDÉ DE STABILISATION DE LA CHARGE D'ALIMENTATION EN GLUCOSE DANS LA PRODUCTION DE GLYCOLS

(30) Priority: 19.12.2016 US 201662436065 P
(43) Date of publication of application: 23.10.2019
(73) Proprietor: Technip Energies France SAS, 92741 Nanterre Cedex (FR)
(72) Inventor: MUTHUSAMY, Duraisamy, Houston Texas 77094 (US)
(74) Representative: Edson, Russell Gregory
(86) International application number: PCT/EP2017/083348
(87) International publication number: WO 2018/114822

(56) References cited:
- WO-A1-2015/154258
- FR-A1- 3 026 407
- US-A1- 2011 313 210
- US-A1- 2015 057 469
- US-A1- 2021 087 127
- GANG XU ET AL: "Remarkable effect of extremely dilute H2SO4 on the cellulose conversion to ethylene glycol", APPLIED CATALYSIS A: GENERAL, vol. 502, 3 June 2015 (2015-06-03), AMSTERDAM, NL, pages 65 - 70, XP055452817, ISSN: 0926-860X, DOI: 10.1016/j.apcata.2015.05.038
- ZHICHAO TAN ET AL: "Catalytic conversion of glucose into alkanediols over nickel-based catalysts: a mechanism study", RSC ADV., vol. 6, no. 67, 22 June 2016 (2016-06-22), pages 62747 - 62753, XP055358943, DOI: 10.1039/C6RA14738K

## Description

The present application claims the benefit of pending United States Patent Application Serial No. 62/436,065, filed December 19, 2016.

### Field of the Invention

The present invention relates to a process for converting a carbohydrate feed stock into glycols. More specifically, the present invention relates to a process for preparing glycols, particularly ethylene glycol and propylene glycol, by converting a carbohydrate feed stock material in a reactor using a bi-functional catalyst system. The process includes the addition of an acid to adjust the pH of the feed solution including the carbohydrate feed and a retro-Aldol catalyst.

### Background

Glycols such as ethylene glycol and propylene glycol are valuable materials with a multitude of commercial applications, e.g. as heat transfer media, antifreeze, and precursors to polymers, such as PET. The market for ethylene and propylene glycols (EG and PG) is expanding worldwide, with the EG market being vastly bigger than the market for PG (i.e., 1,2-propylene glycol). Ethylene and propylene glycols are typically made on an industrial scale by hydrolysis of the corresponding alkylene oxides, which are the oxidation products of ethylene and propylene, produced from fossil fuels/petrochemical feed stocks involving multiple processing steps. Use of bio-based feed stocks for the production of energy and chemicals has become increasingly desirable in the industry since this approach to use feeds from renewable sources provides a pathway for sustainable development.

In recent years, increased efforts have focused on producing chemicals, including glycols, from renewable feed stocks, such as carbohydrate-containing feedstock. Carbohydrates are plentiful and renewable bio-mass feeds having the structural features resembling that of ethylene glycol; each carbon has one attached hydroxyl group or contains an oxygen function that can be readily converted into a hydroxyl. As such, EG and PG can be produced if the C-C bonds are selectively cleaved into C2 and C3 units.

WO2015/154258 A1 describes a catalytic process for the preparation ethylene glycol from a saccharide containing feedstock. More specifically, the invention relates to a process for the selective conversion of saccharide to ethylene glycol using a catalyst system under acidic conditions. US 2015/057469 A1 describes a method for producing glycols from cellulosic materials, and more particularly to a method for catalytically producing glycols from cellulosic materials with ternary catalysts under hydrothermal conditions. US 2011/313210 A1 describes a continuous process for generating at least one polyol from a cellulose containing feedstock. GANG XU ET AL: "Remarkable effect of extremely dilute H2SO4 on the cellulose conversion to ethylene glycol", Applied Catalysis A: General, vol. 502, 1 August 2015 (2015-08-01), pages 65-70 describes the effect of dilute H₂SO₄ cellulose conversion to ethylene glycol.

As with many chemical processes, the reaction product stream in these processes comprises a number of desired materials as well as diluents, by-products and other undesirable materials. In order to provide a high value process, the desirable product or products must be obtainable from the reaction product stream in high purity with a high percentage recovery of each product and with as low as possible use of energy, chemical components and complex equipment. In addition, the process should allow for the selective formation of ethylene glycol over the other glycols, high yields of the total glycols mixture, use of a high-concentration sugar solution as feed to the reactor, and maintain stable catalyst activity over time. In addition, it is desirable to feed both the carbohydrate feed and catalyst together in a single feed to the reactor. These desirable features are challenging to achieve, particularly considering the instability of the carbohydrate feed under high pH conditions resulting from the combined feeding of the carbohydrate and retro-Aldol catalyst.

Therefore, it would be advantageous to provide an improved method suitable for the production of glycols from carbohydrate feeds including a technique to stabilize the carbohydrate feed when in the presence of a retro-Aldol catalyst in order to maintain the desired pH of the carbohydrate feed to the reactor. This would make the overall glycol production process more efficient and economical than processes disclosed previously in the industry.

### Summary of the Invention

According to an embodiment of the disclosed subject matter, methods for producing ethylene glycol from a carbohydrate feed may include preparing a feed solution including the carbohydrate feed, a soluble retro-Aldol catalyst, and an acid. The feed solution may be contacted, in a reactor under hydrogenation conditions, with a heterogeneous hydrogenation catalyst. A product stream including ethylene glycol may be obtained from the reactor.

Implementations of the disclosed subject matter provide an improved method for producing ethylene glycol from a carbohydrate feed. Because the disclosed subject matter avoids the isomerization of glucose to fructose in the presence of a retro-Aldol catalyst and maintains a desired pH of the feed solution including the carbohydrate feed, the process results in: the selective formation of ethylene glycol over the other glycols; high yields of the total glycols mixture; and the ability to use a high-concentration sugar solution as feed to the reactor, all while maintaining stable feed solution and catalyst activity over time. Therefore, the disclosed subject matter provides an improved method suitable for the production of glycols from carbohydrate feeds including a technique for controlling the pH and glucose stability of the feed solution in order to make the overall glycol production process more economical than processes disclosed previously in the industry.

Additional features, advantages, and embodiments of the disclosed subject matter may be set forth or apparent from consideration of the following detailed description, drawings, and claims. Moreover, it is to be understood that both the foregoing summary and the following detailed description are examples and are intended to provide further explanation without limiting the scope of the claims.

### Brief Description of the Drawings

The accompanying drawings, which are included to provide a further understanding of the disclosed subject matter, are incorporated in and constitute a part of this specification. The drawings also illustrate embodiments of the disclosed subject matter and together with the detailed description serve to explain the principles of embodiments of the disclosed subject matter. No attempt is made to show structural details in more detail than may be necessary for a fundamental understanding of the disclosed subject matter and various ways in which it may be practiced.

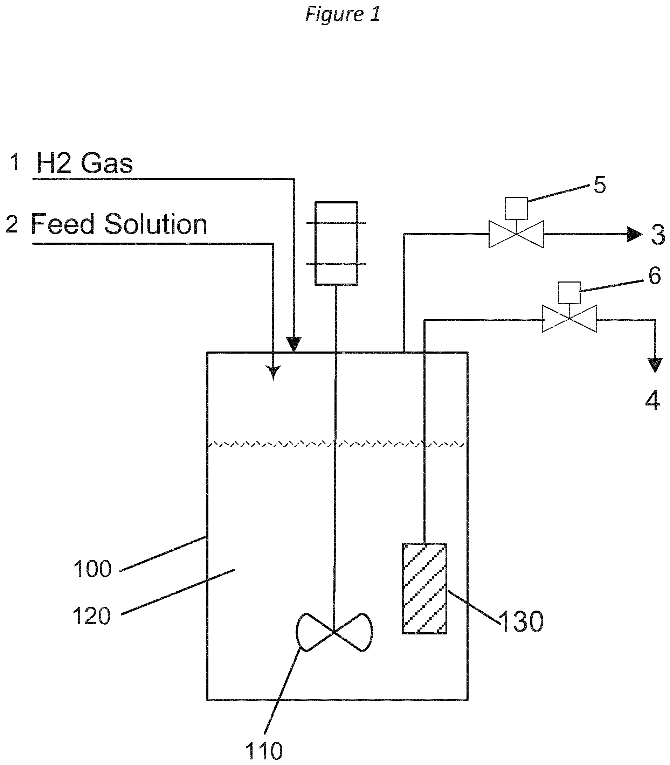

FIG. 1 shows an example process scheme according to an implementation of the disclosed subject matter.

### Detailed Description

Carbohydrates are readily available and renewable bio-mass feeds, and they have the structural features resembling that of ethylene glycol; each carbon has one attached hydroxyl group or contains an oxygen function that can be readily converted into a hydroxyl. Ethylene glycol (EG) and propylene glycol (PG) can be produced by selectively cleaving the C-C bonds into C2 and C3 units. As such, the presently disclosed subject matter provides a process for the conversion of carbohydrate feed stock materials and hydrogen gas into glycols, particularly with ethylene glycol as the main product and propylene glycol as a smaller co-product.

The process variables have major impacts on the conversion and selectivity of the reaction. For example, the particular catalyst(s) used and process conditions can provide for a successful reaction selectivity outcome under a set of practical reaction conditions. Examples of process variables include feed stock (e.g., sucrose, glucose, sorbitol, C5 versus C6 sugars, starch, and the like); stability of the feedstock; one or more catalysts (e.g., having retro-Aldol and hydrogenation functions); temperature, catalyst performance and stability, H2 partial pressure, H2/feed ratio, residence time, reaction medium (e.g., a solvent such as water), pH in the reaction medium, and feed/solvent ratio. According to the presently disclosed subject matter, the stability of the feed solution is identified as being particularly important taking into consideration the chemistry of the reaction discussed below.

The sugars to glycols hydrogenolysis reaction, which is carried out using a metal catalyst and in the presence of hydrogen, is a complex reaction known to produce hundreds of products. Since ethylene and propylene glycols are the desired products, the other products must be minimized by selecting the appropriate catalyst and conditions; additionally an EG/PG wt% ratio of at least 1:1 and preferably 7:1 or more is desirable. In general, sugars tend to cleave into C3 fragments more easily than the desired C2 fragment, resulting in the formation of propylene glycol as the single most predominant molecule. While the selection of the most appropriate catalyst, not only from the selectivity point of view but also from the point of view of catalyst longevity, is an important task, other aspects of the reaction must also be considered. The catalyst generally only controls the chemistry taking place on its surface; for example, the cleavage of the sugar molecules into smaller fragments taking place by discrete retro-Aldol reactions followed by hydrogenation of the intermediates into products is the desired pathway. However, quite a number of other reactions take place in solution and these side reactions must also be considered. A number of ions such as OH-, OAc-, etc. could be present in the solution under basic pH conditions or H+ ions could be present under acidic pH conditions. While these ions could also catalyze the retro-Aldol reaction, these ions are generally known to catalyze a variety of dehydration side-reactions causing the sugar molecules to degrade into wasteful products. These undesirable side reactions could become dominant particularly under high temperature conditions. A proper choice of catalysts and process conditions is therefore essential in order to realize the objectives of high glycol yields and long catalyst life. Multiple equations can be used to explain the various steps of the chemistry of the conversion of sugars to EG and PG, as shown below.

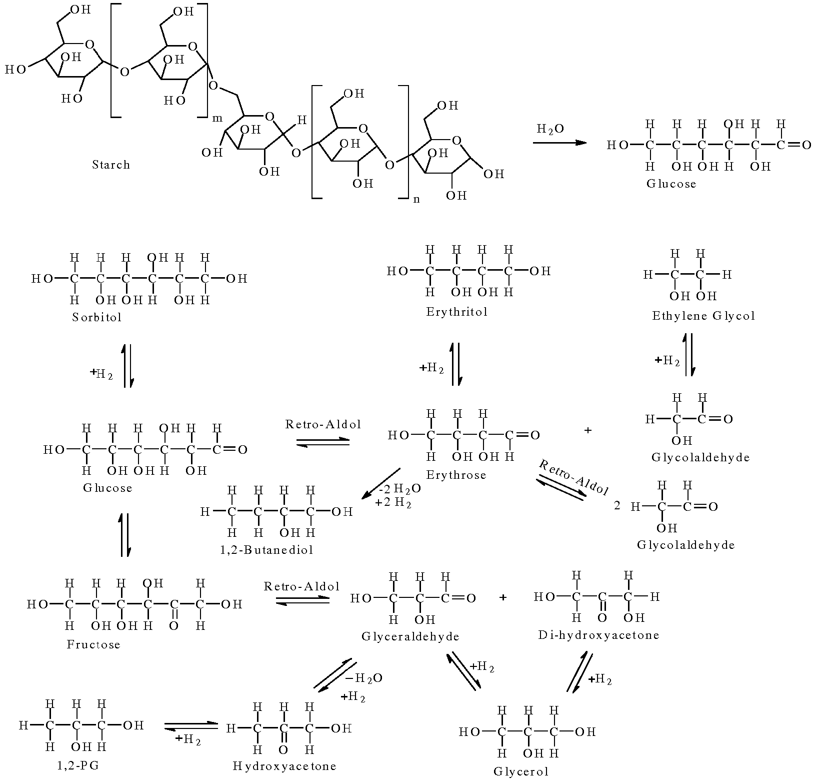

As shown above, the chemistry of sugars in the hydrogenolysis reaction is a notoriously complex set of functional group chemistries; the products from any reaction could be reactants for all other reactions, including those taking place on the surface of the solid catalyst. The product distribution (EG, PG, partially converted sugars, etc.) at the end of reaction will be a function of the relative rates of these reactions under the chosen experimental conditions. Loss of glucose to fructose by the isomerization pathway under basic pH conditions in the feed solution is detrimental to the glycol yields in the carbohydrates-to-glycols process. The isomerization reaction is shown by the equilibrium arrow between the glucose and fructose structures shown in the scheme above. The presently disclose subject matter stops this isomerization reaction by adjusting the pH to an acid side pH. Retro-Aldol cleavage of fructose leads exclusively to C3 products such as glycerol and 1,2-propanediol. Formation of glycerol is undesirable and formation of 1,2-propanediol is less desirable. Ethylene glycol (EG) is the most desirable product and it is therefore of interest to stop yield losses resulting from the instability of glucose in the feed storage vessel. Thus, according to the presently disclosed subject matter, important process variables including catalyst performance and feed stability have been improved for the disclosed method for producing ethylene glycol from a carbohydrate feed.

The presently disclosed method for producing ethylene glycol from a carbohydrate feed has numerous advantages over the prior art. Because the disclosed subject matter avoids the destabilization of glucose to fructose in the presence of a retro-Aldol catalyst and maintains a desired pH of the feed solution including the carbohydrate feed, the process results in: the selective formation of ethylene glycol over the other glycols; high yields of the total glycols mixture; and the ability to use a high-concentration sugar solution as feed to the reactor, all while maintaining stable feed solution and catalyst activity over time.

The disclosed subject matter provides an improved method suitable for the production of glycols from carbohydrate feeds including a technique for controlling the pH and glucose stability of the feed solution in order to make the overall glycol production process more economical than processes disclosed previously in the industry.

According to an implementation of the disclosed subject matter, a method for producing ethylene glycol from a carbohydrate feed may include preparing a feed solution including the carbohydrate feed, a soluble retro-Aldol catalyst, and an acid. The feed solution may be contacted, in a reactor under hydrogenation conditions, with a heterogeneous hydrogenation catalyst. A product stream including ethylene glycol may be obtained from the reactor.

The carbohydrate feed for the process may include one or more of glucose, sucrose, xylose, sugar cane molasses, starch (e.g., hydrolyzed starch, corn syrup, and the like), and cellulose (e.g., hydrolyzed cellulose, and the like). In an embodiment, the carbohydrate feed may include corn syrup comprising glucose as the preferred feed. In an embodiment, the carbohydrate feed may include a concentration of carbohydrate, in the total solution entering the reactor of 5-40 wt% in a solvent, at least 5 wt% in a solvent, and at least 10 wt% in a solvent.

The feed solution may include the carbohydrate feed, a soluble retro-Aldol catalyst, and an acid. The feed solution may include a glucose content and a fructose content. In an implementation, the fructose content may be less than 1% of the glucose content.

The acid in the feed solution may be one of a low-molecular weight organic acid, carbonic acid, a mineral acid, and combinations thereof. The low-molecular weight organic acid may be at least one of: formic acid, acetic acid, propionic acid, butyric acid, glycolic acid, lactic acid, citric acid, benzoic acid, oxalic acid, and combinations thereof. In an embodiment, the low-molecular weight organic acid may be one of: acetic acid, lactic acid, glycolic acid, and combinations thereof. The mineral acid may be at least one of: sulfuric acid, phosphoric acid, boric acid, and combinations thereof.

In an aspect, the pH of the feed solution in step (a) is maintained in the range from 2-6. The pH of the feed solution is controlled and/or maintained to be in the range of from 2-6 based on the addition of acid to the feed solution. The concentration of the acid in step (a) may be based on the concentration of the soluble retro-Aldol catalyst, in the total solution entering the first reactor. For example, the concentration of the acid in the feed solution is adjusted in order to maintain the pH of the feed solution to be within the desired pH range of from 2-6. The acid concentration used to achieve the desired pH in the pH range of 2 to 6 may be based on the concentration and type of the tungstate retro-Aldol catalyst present in the carbohydrate feed solution.

The soluble retro-Aldol catalyst may comprise one or more compounds, complex or elemental material comprising tungsten, molybdenum, vanadium, niobium, chromium, titanium or zirconium. In particular, the soluble retro-Aldol catalyst may comprise one or more material selected from the list consisting of tungstic acid, molybdic acid, ammonium tungstate, ammonium metatungstate, ammonium paratungstate, tungstate compounds comprising at least one Group I or II element, metatungstate compounds comprising at least one Group I or II element, paratungstate compounds comprising at least one Group I or II element, heteropoly compounds of tungsten, heteropoly compounds of molybdenum, tungsten oxides, molybdenum oxides, vanadium oxides, metavanadates, chromium oxides, chromium sulfate, titanium ethoxide, zirconium acetate, zirconium carbonate, zirconium hydroxide, niobium oxides, niobium ethoxide, and combinations thereof. The metal component is in a form other than a carbide, nitride, or phosphide. According to an embodiment, the soluble retro-Aldol catalyst includes at least one of:
silver tungstate, sodium meta-tungstate, ammonium meta-tungstate, sodium poly-tungstate, tungstic acid, alkali- and alkaline-earth metal tungstates, sodium phospho-tungstate, phospho-tungstic acid, alkali- and alkaline-earth metal phospho-tungstates, alkali- and alkaline-earth metal molybdates, alkali- and alkaline-earth metal phospho-molybdates, phospho-molybdic acid, heteropoly acids, mixed tungstates and molybdates, niobic acid, silicotungstic acid, alkali- and alkaline-earth metal niobates. In an aspect, the soluble retro-Aldol catalyst may be sodium tungstate.

Examples of heterogeneous hydrogenation catalysts are supported and un-supported metal catalysts selected from Group 8 to Group 11 metals in the periodic table. Examples of un-supported metal catalysts are Raney-metal catalysts such as Raney-Ni, Raney-Co, Raney-Cu, and Raney-Ru, and metal-powder catalysts such as powdered Ni, Co, Cu, Cu-Zn, Cu-Cr, Ni-Mo, Ni-W, and Ni-Cr. The heterogeneous hydrogenation catalyst may be promoted with metals such as Al, Fe, Cr, Mn, Co, Cu, Mo, Ru, Rh, Pd, Ag, W, Re, Ir, Pt, Au, In, Sn, Sb, and Pb. One or more metals may be used in the formulation of the promoted metal catalysts. The promoting metals may be present in concentrations ranging from about 0.001 wt% to about 10 wt%. Examples of supported-metal hydrogenation catalysts are Group 8 to Group 11 metal catalysts supported on hydrothermally stable supports such as TiO2, ZrO2, and alpha-alumina. The metals may be used individually or in combination with one or more of the other metals.

According to an embodiment, at least one of the heterogeneous hydrogenation catalyst and soluble retro-Aldol catalyst is supported on a solid support. In an embodiment, any other active catalyst component may be present in either heterogeneous or homogeneous form. In this case, any other active catalyst component may also be supported on a solid support. In one embodiment, the heterogeneous hydrogenation catalyst is supported on one solid support and the soluble retro-Aldol catalyst is supported on a second solid support which may comprise the same or different material. As a specific example, the heterogeneous hydrogenation catalyst may be a hydrogenation catalyst supported on a hydrothermally stable support. In another embodiment, both the heterogeneous hydrogenation catalyst and soluble retro-Aldol catalyst are supported on one solid hydrothermally stable support.

The solid support may be in the form of a powder or in the form of regular or irregular shapes such as spheres, extrudates, pills, pellets, tablets, monolithic structures. Alternatively, the solid supports may be present as surface coatings, for examples on the surfaces of tubes or heat exchangers. Suitable solid support materials are those known to the skilled person and include, but are not limited to aluminas, silicas, zirconium oxide, magnesium oxide, zinc oxide, titanium oxide, carbon, activated carbon, zeolites, clays, silica alumina and mixtures thereof.

The presently disclosed process may also include a reaction solvent. The reaction solvent may be water, a C1 to C6 alcohol, a C1 to C6 polyol, or mixtures thereof. Further solvent may also be added to the reactor in a separate feed stream or may be added to the carbohydrate feed before it enters the reactor. Examples of C1 to C6 polyols include 1,2-hexanediol, glycerol, etc. As an example, the reaction solvent may be a mixture including H2O and at least one of alcohols, ethers, and ether-alcohols, and mixtures thereof. In an embodiment, the reaction solvent may be H2O.

Suitable reactor vessels to be used in the process of the preparation of ethylene glycol from a carbohydrate feed include continuous stirred tank reactors (CSTR), plug-flow reactors, slurry reactors, ebbulated bed reactors, jet flow reactors, mechanically agitated reactors, back-mixed reactors, bubble columns, such as slurry bubble columns and external recycle loop reactors. The use of these reactor vessels allows dilution of the reaction mixture to an extent that provides high degrees of selectivity to the desired glycol product (mainly ethylene and propylene glycols). There may be one or more of such reactor vessels, arranged in series. In one embodiment, preferably there are two reactor vessels arranged in series, the first one of which is a CSTR, the output of which is supplied into a plug-flow reactor.

The disclosed method for producing ethylene glycol from a carbohydrate feed may be performed under particular hydrogenation conditions in order to maximize the desired yield of EG. For example, the hydrogenation conditions may include temperature, pressure, flow rate, and any other process variable that may be controlled. In an embodiment, the hydrogenation conditions may include a temperature in the range of from 180-250°C and from 210-250°C. The hydrogenation conditions may also include a pressure in the range of from 3.44 to 13.79 MPag (500 to 2000 psig).

In an embodiment, the presently disclosed method may also include contacting the carbohydrate feed with hydrogen. For example, the disclosed method may take place in the presence of hydrogen. Hydrogen may be supplied into the reactor vessel under pressure in a manner common in the art. Hydrogen is supplied into the reactor vessels under pressure. In an example, the method of the present reaction takes place in the absence of air or oxygen. In order to achieve this, it is preferable that the atmosphere in the reactor vessel be evacuated and replaced with hydrogen repeatedly, after loading of any initial reactor vessel contents, before the reaction starts.

In an embodiment, the disclosed method may also include running the reaction under pH controlled conditions in the reactor. In particular, the pH of the reaction may be in the range of from 2-6. This pH may be the pH of the feed solution entering the reactor and/or the pH inside the reactor. The pH of the feed solution may be controlled and/or maintained to be in the range of from 2-6 based on the addition of acid in the feed solution. The concentration of the acid in step (a) may be based on the concentration of the soluble retro-Aldol catalyst, in the total solution entering the first reactor. The pH may also be controlled using at least one pH controlling agent such as alkali- and alkaline-earth metal salts of carbonic acid or carboxylic acids or combinations thereof, alkali- and alkaline-earth metal salts of phosphoric acid, zinc carbonate, and zinc salts of carboxylic acids.

According to the presently disclosed subject matter, a product stream may be obtained from the reactor including ethylene glycol. The product stream may include at least 5 wt% concentration of glycols. In addition, the product stream may include a yield of at least 60 wt% glycols, and at least 70 wt% glycols. In an embodiment, the product stream may include a yield of at least 60 wt% EG, and at least 65 wt% EG. An advantage of the presently disclosed method is the ability to maximize the yield of EG relative to the yield of PG. For example, the product stream may include an EG/PG wt% yield ratio of at least 1:1, a EG/PG wt% yield ratio of at least 7:1, and a EG/PG wt% yield ratio of at least 10:1. In addition, the presently disclosed method allows for minimizing undesired products of the subject reaction. Accordingly, the product stream may include a yield of no more than 10 wt% sorbitol. Further, the product stream may include a yield of less than 3 wt% 1,2-butanediol. Additionally, the product stream may include a minimum EG/1,2BDO wt% yield ratio of 20:1, thereby maximizing the EG yield relative to other less desired products.

According to an embodiment, the product stream may be further processed. For example, the product stream may be fed to a second reactor which may include contacting the product stream from the first reactor with hydrogen in the presence of a heterogeneous hydrogenation catalyst. A final product stream comprising ethylene glycol may be obtained that is substantially free of compounds containing carbonyl functional groups. The heterogeneous hydrogenation catalyst used in this further processing of the product stream may or may not be the same heterogeneous hydrogenation catalyst used in the bi-functional catalyst system in the glycols production process.

FIG. 1 shows an example process scheme according to an implementation of the disclosed subject matter. An example apparatus and scheme that may be used to perform the conversion of carbohydrate feeds into glycols using a catalyst system comprising a heterogeneous hydrogenation catalyst and a homogeneous tungstate retro-Aldol catalyst are schematically represented in Figure 1. As shown in FIG. 1, reactor 100 may be equipped with stirrer 110 and catalyst filter 130. The reactor may also be equipped with automatic controls for the control of reactor temperature, back-pressure, liquid holdup level, and stirrer speed. The feed line 1 may be equipped with a gas flowmeter and may be used to provide a continuous flow of hydrogen gas into the reactor 100. The feed line 2, which may be equipped with a pump and a mass flow meter, may be used to send the feed solution containing the carbohydrate feed, the tungstate retro-Aldol catalyst, and the acid (added to adjust the pH of the solution) to the reactor 100. Typically, the heterogeneous hydrogenation catalyst may be charged to the reactor 100 at the beginning of the reactor operation. The filter element 130 may be used to retain the heterogeneous hydrogenation catalyst and any precipitated oxides of tungsten (W-oxides) present in the reaction medium 120. The excess gas present in the reactor 100 may be vented via vent gas stream 3 by the use of the back-pressure control valve 5. The liquid product stream flowing out of the reactor is stream 4 and is controlled by valve 6.

In the disclosed method for the preparation of ethylene glycol from a carbohydrate-containing feed, the residence time in the reactor vessel of the reaction mixture may be at least 1 minute, at least 2 minutes, and at least 5 minutes. Suitably the residence time in the reactor vessel is no more than 5 hours, no more than 2 hours, and no more than 1 hour. According to an implementation, the average residence time in the reactor is no more than 2 hours.

As shown in the Examples section provided below, the presently disclosed method for producing ethylene glycol from a carbohydrate feed has numerous advantages over the prior art. Because the disclosed subject matter avoids the destabilization of glucose to fructose in the presence of a retro-Aldol catalyst and maintains a desired pH of the carbohydrate feed, the process results in the selective formation of ethylene glycol over the other glycols, high yields of the total glycols mixture, and the ability to use a high-concentration sugar solution as feed to the reactor, all while maintaining stable feed solution and catalyst activity over time. Therefore, the disclosed subject matter provides an improved method suitable for the production of glycols from carbohydrate feeds including a technique for controlling the pH and stability of glucose in the feed solution in order to make the overall glycol production process more economical than processes disclosed previously in the industry.

### EXAMPLES

In Comparative Examples 1 and 2, feed solutions containing a glucose feed and sodium tungstate retro-Aldol catalyst were prepared in deionized water and the pH of the solution was measured. The solutions were allowed to stand at room temperature for varying amounts of time and analyzed by high pressure liquid chromatography (HPLC) to determine the composition of the sugar feed. The results are provided in Table 1 below.

In Examples 3 to 6, feed solutions containing the glucose feed and sodium tungstate retro-Aldol catalyst were prepared. Specified amounts of glacial acetic acid were added to these solutions in order to adjust the pH of the solutions. The solutions were allowed to stand at room temperature for varying amounts of time and analyzed by HPLC to determine the composition of the sugar feed. The results are provided in Table 1 below.

In Examples 7 to 9, feed solutions containing the glucose feed and sodium tungstate retro-Aldol catalyst were prepared. Specified amounts of lactic acid were added to these solutions in order to adjust the pH of the solutions. The solutions were allowed to stand at room temperature for varying amounts of time and analyzed by HPLC to determine the composition of the sugar feed. The results are provided in Table 1 below.

In Example 10, a solution containing the glucose feed and sodium tungstate retro-Aldol catalyst was prepared. The solution was acidified by the addition of 5N H2SO4 solution in order to adjust the pH of the solution. The solution was allowed to stand at room temperature for a period of 50 hours and then analyzed by HPLC to determine the composition of the sugar feed. The results are provided in Table 1 below. The concentration of H2SO4 shown in Table 1 is based on 100% H2SO4.

**Table 1: Examples Showing the Stabilization of Glucose by pH Adjustment**

| Examples | Storage Time | Weight % Composition of Glucose Feed Solution* | | | | | | |
|---|---|---|---|---|---|---|---|---|
| | [Hours] | Glucose | Fructose | Sodium Tungstate ** | Acetic Acid | Lactic Acid | H2SO4 | pH |
| Example 1 | 0 | 48.0 | 0 | 2.1 | | | | 9.0 |
| | 99 | 45.2 | 2.8 | 2.1 | | | | |
| | | | | | | | | |
| Example 2 | 0 | 40.0 | 0 | 2.1 | | | | 8.6 |
| | 47 | 39.5 | 0.5 | 2.1 | | | | |
| | 211 | 38.9 | 1.1 | 2.1 | | | | |
| | | | | | | | | |
| Example 3 | 0 | 40.0 | 0 | 2.1 | 0.5 | | | 5.3 |
| | 47 | 39.7 | 0 | 2.1 | 0.8 | | | |
| | 211 | 39.7 | 0 | 2.1 | 0.8 | | | |
| | | | | | | | | |
| Example 4 | 0 | 40.0 | 0 | 2.0 | 1.0 | | | 4.5 |
| | 47 | 39.7 | 0 | 2.0 | 1.3 | | | |
| | 211 | 39.7 | 0 | 2.0 | 1.3 | | | |
| | | | | | | | | |
| Example 5 | 0 | 40.0 | 0 | 2.0 | 2.0 | | | 4.0 |
| | 47 | 39.7 | 0 | 2.0 | 2.3 | | | |
| | 211 | 39.7 | 0 | 2.0 | 2.3 | | | |
| | | | | | | | | |
| Example 6 | 0 | 46.3 | 0 | 2.0 | 3.5 | | | 3.5 |
| | 67 | 46.1 | 0 | 2.0 | 3.7 | | | |
| | | | | | | | | |
| Example 7 | 0 | 40.0 | 0 | 2.0 | | 0.5 | | 7.4 |
| | 47 | 39.2 | 0.5 | 2.0 | | 0.8 | | |
| | 211 | 39.1 | 0.6 | 2.0 | | 0.8 | | |
| | | | | | | | | |
| Example 8 | 0 | 40.0 | 0 | 2.0 | | 1.0 | | 6.1 |
| | 47 | 39.7 | 0 | 2.0 | | 1.3 | | |
| | 211 | 39.7 | 0 | 2.0 | | 1.3 | | |
| | | | | | | | | |
| Example 9 | 0 | 40.0 | 0 | 2.0 | | 2.0 | | 2.2 |
| | 47 | 39.7 | 0 | 2.0 | | 2.3 | | |
| | 211 | 39.6 | 0 | 2.0 | | 2.5 | | |
| | | | | | | | | |
| Example 10 | 0 | 46.9 | 0 | 2.1 | | | 0.44 | 3.5 |
| | 50 | 46.9 | 0 | 2.1 | | | 0.44 | |

| | | | | | | | | |
|---|---|---|---|---|---|---|---|---|
| * Compositions reported are based on weights used to prepare the solutions or normalized HPLC results; Glucose, fructose, acetic acid, and lactic acid are reported from HPLC analysis. **Sodium tungstate = Na2WO4.2H2O | | | | | | | | |

As can be seen from the results shown in Table 1 above, the pH of the glucose feed solution containing the sodium tungstate retro-Aldol catalyst is on the basic side and the pH ranged from 8.6 to 9. Glucose is found to be unstable under these basic pH conditions; it isomerizes to fructose. In Comparative Example 1, which contained 48% weight glucose and 2.1% wt sodium tungstate, approximately 6% of the glucose had isomerized to fructose during a period of 99 hours. In Comparative Example 2, which contained 40% weight glucose and 2.1% wt sodium tungstate, approximately 3% of the glucose had isomerized to fructose during a period of 211 hours. In Example 7, which contained 40% wt glucose and 2.0% wt sodium tungstate at a pH of 7.4, 1.4% of the glucose had isomerized to fructose over a period of 211 hours. Although lactic acid was used to lower the pH of the glucose solution in Example 7, the pH of 7.4 (i.e., outside of the desired pH range of 2-6) is still high enough to catalyze the glucose to fructose isomerization reaction, demonstrating the need to control the pH in the desirable range of 2-6 which is also dependent upon the amount of the acid used. In all other Examples, namely Examples 3-6 and 8-10, the glucose feed was completely stable and no fructose was observed in the HPLC chromatogram. Acetic acid was used to adjust the pH in Examples 3 to 6, lactic acid was used in Examples 8 and 9, and sulfuric acid was used in Example 10. These Examples show that any low-molecular weight organic acid such as acetic acid, lactic acid, or sulfuric acid can be used to adjust the pH of the feed solution containing the glucose feed and the sodium tungstate retro-Aldol catalyst, and glucose is stable at room temperature in the pH window of 2 to 6. The amount of acid (e.g., concentration of acid) used to adjust the pH at the desired level may be based on the concentration and type of tungstate present in the carbohydrate feed solution.

As shown in the Examples section above, the presently disclosed method for producing ethylene glycol from a carbohydrate feed has numerous advantages over the prior art. Loss of glucose to fructose by the isomerization pathway is detrimental to the glycol yields in the carbohydrates-to-glycols process. Retro-Aldol cleavage of fructose leads exclusively to C3 products such as glycerol and 1,2-propanediol. Formation of glycerol is undesirable and formation of 1,2-propanediol is less desirable. Ethylene glycol (EG) is the most desirable product and it is therefore a feature of the presently disclosed subject matter to stop yield losses resulting from the instability of glucose in the feed storage vessel. Because the disclosed subject matter avoids the destabilization of glucose to fructose in the presence of a retro-Aldol catalyst and maintains a desired pH of the feed solution including the carbohydrate feed, the process results in the selective formation of ethylene glycol over the other glycols, high yields of the total glycols mixture, and the ability to use a high-concentration sugar solution as feed to the reactor, all while maintaining stable feed solution and catalyst activity over time. Therefore, the disclosed subject matter provides an improved method suitable for the production of glycols from carbohydrate feeds including a technique for controlling the pH and glucose stability in the feed solution in order to make the overall glycol production process more economical than processes disclosed previously in the industry.

The foregoing description, for purpose of explanation, has been described with reference to specific embodiments. However, the illustrative discussions above are not intended to be exhaustive or to limit embodiments of the disclosed subject matter to the precise forms disclosed. Many modifications and variations are possible in view of the above teachings. The embodiments were chosen and described in order to explain the principles of embodiments of the disclosed subject matter and their practical applications, to thereby enable others skilled in the art to utilize those embodiments as well as various embodiments with various modifications as may be suited to the particular use contemplated.

## Claims

1. A method for producing ethylene glycol from a carbohydrate feed comprising:
a) preparing a feed solution comprising the carbohydrate feed, a soluble retro-Aldol catalyst, and an acid, wherein the pH of the feed solution is maintained in the range from 2-6;
b) contacting, in a reactor under hydrogenation conditions, the feed solution with a heterogeneous hydrogenation catalyst, and
c) obtaining a product stream, from the first reactor, comprising ethylene glycol, and
wherein the soluble retro-Aldol catalyst comprises at least one selected from the group consisting of: silver tungstate, sodium meta-tungstate, ammonium meta-tungstate, sodium poly-tungstate, tungstic acid, alkali- and alkaline-earth metal tungstates, sodium phospho-tungstate, phospho-tungstic acid, alkali- and alkaline-earth metal phospho-tungstates, alkali- and alkaline-earth metal molybdates, alkali- and alkaline-earth metal phospho-molybdates, phospho-molybdic acid, heteropoly acids, mixed tungstates and molybdates, niobic acid, silicotungstic acid, alkali- and alkaline-earth metal niobates.

2. The method of claim 1, wherein the feed solution comprises a glucose content and a fructose content, and wherein the fructose content is less than 1% of the glucose content.

3. The method of claim 1, wherein the acid is at least one selected from the group consisting of: a low-molecular weight organic acid, carbonic acid, a mineral acid, and combinations thereof.

4. The method of claim 3, wherein the low-molecular weight organic acid is at least one selected from the group consisting of: formic acid, acetic acid, propionic acid, butyric acid, glycolic acid, lactic acid, citric acid, benzoic acid, oxalic acid, and combinations thereof.

5. The method of claim 3, wherein the low-molecular weight organic acid is at least one selected from the group consisting of: acetic acid, lactic acid, glycolic acid, and combinations thereof.

6. The method of claim 3, wherein the mineral acid is at least one selected from the group consisting of: sulfuric acid, phosphoric acid, and boric acid.

7. The method of claim 1, wherein the soluble retro-Aldol catalyst comprises sodium tungstate.

## Patentansprüche

1. Verfahren zum Herstellen von Ethylenglykol aus einer Kohlenhydratzufuhr, umfassend:
a) Herstellen einer Zufuhrlösung, umfassend die Kohlenhydratzufuhr, einen löslichen Retro-Aldol-Katalysator und eine Säure, wobei der pH-Wert der Zufuhrlösung in dem Bereich von 2-6 gehalten wird;
b) Inkontaktbringen, in einem Reaktor unter Hydrierungsbedingungen, der Zufuhrlösung mit einem heterogenen Hydrierungskatalysator und
c) Erhalten eines Produktstroms aus dem ersten Reaktor, umfassend Ethylenglykol, und
wobei der lösliche Retro-Aldol-Katalysator mindestens eines umfasst, ausgewählt aus der Gruppe, bestehend aus: Silberwolframat, Natriummetawolframat, Ammoniummetawolframat, Natriumpolywolframat, Wolframsäure, Alkali- und Erdalkalimetallwolframaten, Natriumphosphowolframat, Phosphowolframsäure, Alkali- und Erdalkalimetallphosphowolframaten, Alkali- und Erdalkalimetallmolybdaten, Alkali- und Erdalkalimetallphosphomolybdaten, Phosphomolybdänsäure, Heteropolysäuren, gemischten Wolframaten und Molybdaten, Niobsäure, Kieselwolframsäure, Alkali- und Erdalkalimetallniobaten.

2. Verfahren nach Anspruch 1, wobei die Zufuhrlösung einen Glukosegehalt und einen Fruktosegehalt umfasst und wobei der Fruktosegehalt weniger als 1 % des Glukosegehalts beträgt.

3. Verfahren nach Anspruch 1, wobei die Säure mindestens eine ist, ausgewählt aus der Gruppe, bestehend aus: einer organischen Säure mit niedrigem Molekulargewicht, Kohlensäure, einer Mineralsäure und Kombinationen davon.

4. Verfahren nach Anspruch 3, wobei die organische Säure mit niedrigem Molekulargewicht mindestens eine ist, ausgewählt aus der Gruppe, bestehend aus: Ameisensäure, Essigsäure, Propionsäure, Buttersäure, Glykolsäure, Milchsäure, Zitronensäure, Benzoesäure, Oxalsäure und Kombinationen davon.

5. Verfahren nach Anspruch 3, wobei die organische Säure mit niedrigem Molekulargewicht mindestens eine ist, ausgewählt aus der Gruppe, bestehend aus: Essigsäure, Milchsäure, Glykolsäure und Kombinationen davon.

6. Verfahren nach Anspruch 3, wobei die Mineralsäure mindestens eine ist, ausgewählt aus der Gruppe, bestehend aus: Schwefelsäure, Phosphorsäure und Borsäure.

7. Verfahren nach Anspruch 1, wobei der lösliche Retro-Aldol-Katalysator Natriumwolframat umfasst.

## Revendications

1. Procédé de production d'éthylène glycol à partir d'une alimentation en hydrate de carbone comprenant :
a) la préparation d'une solution d'alimentation comprenant l'alimentation en hydrate de carbone, un catalyseur rétro-Aldol soluble, et un acide, dans lequel le pH de la solution d'alimentation est maintenu dans la plage allant de 2 à 6 ;
b) la mise en contact, dans un réacteur dans des conditions d'hydrogénation, de la solution d'alimentation avec un catalyseur d'hydrogénation hétérogène, et
c) l'obtention d'un courant de produit, à partir du premier réacteur, comprenant de l'éthylène glycol, et
dans lequel le catalyseur rétro-Aldol soluble comprend au moins un catalyseur choisi dans le groupe constitué de : tungstate d'argent, métatungstate de sodium, métatungstate d'ammonium, polytungstate de sodium, acide tungstique, tungstates de métaux alcalins et alcalino-terreux, phosphotungstate de sodium, acide phosphotungstique, phosphotungstates de métaux alcalins et alcalino-terreux, molybdates de métaux alcalins et alcalino-terreux, phosphomolybdates de métaux alcalins et alcalino-terreux, acide phosphomolybdique, hétéropolyacides, tungstates et molybdates mixtes, acide niobique, acide silicotungstique, niobates de métaux alcalins et alcalino-terreux.

2. Procédé selon la revendication 1, dans lequel la solution d'alimentation comprend une teneur en glucose et une teneur en fructose, et dans lequel la teneur en fructose est inférieure à 1 % de la teneur en glucose.

3. Procédé selon la revendication 1, dans lequel l'acide est au moins un acide choisi dans le groupe constitué de : un acide organique de faible masse moléculaire, l'acide carbonique, un acide minéral, et des combinaisons de ceux-ci.

4. Procédé selon la revendication 3, dans lequel l'acide organique de faible masse moléculaire est au moins un acide choisi dans le groupe constitué de : acide formique, acide acétique, acide propionique, acide butyrique, acide glycolique, acide lactique, acide citrique, acide benzoïque, acide oxalique, et des combinaisons de ceux-ci.

5. Procédé selon la revendication 3, dans lequel l'acide organique de faible masse moléculaire est au moins un acide choisi dans le groupe constitué de : acide acétique, acide lactique, acide glycolique, et des combinaisons de ceux-ci.

6. Procédé selon la revendication 3, dans lequel l'acide minéral est au moins un acide choisi dans le groupe constitué de : acide sulfurique, acide phosphorique, et acide borique.

7. Procédé selon la revendication 1, dans lequel le catalyseur rétro-Aldol soluble comprend du tungstate de sodium.
